# EUROPEAN PATENT APPLICATION

(11) **EP 4 005 553 A1**
(43) Date of publication of application: **01.06.2022**
(21) Application number: 20848631.6
(22) Date of filing: 20.01.2020
(51) Int. Cl.: A61K 8/9789, A61K 8/44, A61Q 19/06, A61Q 19/00, A61P 17/04

(54) **EXTERNAL COMPOSITION FOR SKIN HAVING ITCHING RELIEVING EFFECT**

(30) Priority: 30.07.2019 CN 201910694724
(71) Applicant: Yangshengtang (ANJI) Cosmetics Co., Ltd., Huzhou, Zhejiang 313399 (CN)
(72) Inventor: ZHAO, Shizhi, Huzhou, Zhejiang 313399 (CN); WANG, Shasha, Huzhou, Zhejiang 313399 (CN); LI, Ping, Huzhou, Zhejiang 313399 (CN)
(74) Representative: J A Kemp LLP
(86) International application number: PCT/CN2020/073125
(87) International publication number: WO 2021/017448

(57) **Abstract**

The present invention relates to a skin topical composition having an antipruritic efficacy, comprising a combination of (A) birch sap and (B) betaine, and the skin topical composition does not comprise water added as a separate component. The skin topical composition is a pharmaceutical composition or a cosmetic composition.

## Description

### Technical field

The present invention relates to a skin topical composition having an antipruritic efficacy, comprising a combination of (A) birch sap and (B) betaine, and the skin topical composition does not comprise water added as a separate component.

### Background art

With age, the function of human sebaceous glands gradually declines, and the skin's ability to retain moisture begins to decline. When autumn and winter come, many people feel skin itchy. In addition, eczema, psoriasis, dermatitis, dry skin and other skin diseases are also accompanied by itching, which severely affect the patients' health and life quality. If the patients scratch too much, the affected area will be covered with scratches, which will affect the appearance of the skin and even cause skin infection or ulceration. Therefore, it is very necessary to develop a skin topical product that can effectively alleviate the itching can be used for a long time, especially a cosmetic or skin care product that can be used for a long time.

The mechanism of skin itch has not been completely clear at present. Studies in recent years have shown that the occurrence of skin itch may be related to the participation of several mediators, such as histamine, serotonin, neuropeptides, and cytokines. Among them, histamine mainly exists in metachromatic granules in mast cells, and skin blood vessels have H₁ and H₂ receptors, and the vasodilation and increased vascular permeability caused by histamine are due to the two receptors. Histamine causes itching in the epidermis or basement membrane, and causes pain and edema while being released into the dermis. Therefore, inhibiting mast cell activation and degranulation and thereby reducing histamine release can alleviate skin itch.

Traditional Chinese medicine treatment methods have disadvantages, such as inconvenient use, poor transdermal absorption, and unknown treatment mechanism. Hormone products can only be used for a short period of time, and their long-term use has obvious side effects and dependence, so they cannot be used as daily care. In view of the above problems, the present invention provides a skin topical composition, especially a cosmetic composition that can fundamentally inhibit the release of an itch mediator, histamine, and thereby effectively alleviate itching. The composition can be applied to different cosmetic formulations, and has the advantages of convenient use, easy absorption, and long-term use while having good antipruritic efficacy.

### Summary of the invention

In one aspect, the present invention relates to the use of a combination of birch sap and betaine in a skin topical composition having an antipruritic efficacy, wherein the skin topical composition does not comprise water added as a separate component.

In another aspect, the present invention provides a skin topical composition having an antipruritic efficacy, comprising a combination of (A) birch sap and (B) betaine, wherein the skin topical composition does not comprise water added as a separate component.

The skin topical composition includes, but is not limited to, a pharmaceutical composition and a cosmetic composition, especially a cosmetic composition, and more particularly a skin care cosmetic composition.

The skin topical composition according to the present invention can inhibit the release of histamine from mast cells, and therefore has a good effect of alleviating and inhibiting skin itch. Compared to an aqueous system or to an aqueous system comprising birch sap alone or an aqueous system comprising betaine alone, the skin topical composition according to the present invention provides significantly improved antihistamine efficacy, showing that the combination of birch sap and betaine produced synergistic effect in anhydrous systems.

The skin topical composition according to the present invention does not comprise any additionally added water component, but does not exclude inherent moisture in the birch sap used or small or trace amounts of water unavoidably introduced from other ingredients.

In one embodiment, the skin topical composition according to the present invention does not comprise a chelating agent such as EDTA salt, sodium polyphosphate, sodium metaphosphate, and gluconic acid.

The birch trees involved in the present invention belong to Betula Betulaceae, and can be derived from three varieties of Betula alba, Betula Pendula, and Betula platyphylla.

The component (A) birch sap is also called birch juice, white birch sap and the like. Birch sap is rich in nutrients, including sugars, fatty acids, amino acids, mineral elements, etc., its collection time is from the time when the snow begins to melt to the time when the trees foliate. During the collection, a small hole is drilled in the birch trunk 40-60 cm away from the ground, and a liquid guiding device is introduced. The birch sap is collected, filtered, and sterilized by conventional methods to obtain a colorless, transparent and nutritious birch sap without precipitates or impurities. The birch sap is commercially available from Daxinganling Chaoyue Wild Berry Development Co., Ltd.

In the present invention, the birch sap may be used as it is (in this case, also called birch sap stock solution), or may be used in a form of birch sap concentrate (also called concentrated birch sap).

The inventors have found that compared to the combination of non-concentrated birch sap stock solution and betaine, the combination of birch sap concentrate having a concentration degree of 1.05-8 times, preferably 1.1-4 times, more preferably 1.2-2 times and betaine showed better efficacy of alleviating skin itch.

The method for concentrating birch sap is as follows: introducing fresh birch sap stock solution into a reverse osmosis circulation device, controlling operation pressure at 0.5-5 bar, temperature at 20-35°C, then cooling to -65°C, vacuuming to 0.1 Pa, circulating until the birch sap is concentrated to a degree of 1.1 times, 1.2 times, 1.5 times, 2 times, 4 times, 8 times and so on, respectively.

The skin topical composition according to the present invention comprises 5-99.9% by weight, preferably 20-95% by weight, and more preferably 35-90% by weight of birch sap, based on the total weight of the skin topical composition.

The component (B) betaine, also known as trimethylglycine, easily interacts with water and has excellent moisturizing perfomance. The betaine used in the present invention is available from Connell Bros. (Shanghai) Co., Ltd. and so on.

The skin topical composition according to the present invention comprises 0.01-20% by weight, preferably 0.1-15% by weight, and more preferably 1-10% by weight of betaine, based on the total weight of the skin topical composition.

In addition to the above-mentioned components (A) and (B), the skin topical composition according to the present invention may optionally comprise (C) an ingredient commonly used in a pharmaceutical composition or a cosmetic composition, including a vehicle, a surfactant, a skin care active ingredient, a pharmaceutical active ingredient, and an excipient and other ingredients known in the art, and their type and amount can be selected as required. Typically, the content of component (C) is 0-65% by weight, based on the total weight of the skin topical composition.

The vehicle is known in the art and includes, but is not limited to, a diluent, a dispersant, or a carrier. Examples include, but are not limited to, ethanol, butanediol, dipropylene glycol and the like. Those skilled in the art can select their type and amount as required. Typically, the vehicle is present in the skin topical composition of the present invention in an amount of 0.5-20%, based on the total weight of component (C).

The surfactant is any type of surfactants commonly used in medicine or cosmetics for reducing the surface tension of the interface and achieving the purpose of cleaning, emulsifying, and stabilizing the system. Examples of the surfactant include, but are not limited to, one or more of soaps of fatty acids (e.g., as sodium laurate, sodium palmitate, etc.), higher alkyl sulfates (e.g., sodium lauryl sulfate, etc.), N-acylsarcosine (e.g.,sodium lauroyl sarcosinate, etc.), higher fatty acid amide sulfonates (e.g., sodium laurylmethyltaurate, etc.), alkylbenzenesulfonates, higher fatty acid ester sulfates (e.g., hardened coconut oil fatty acid glycerine sodium sulfates, etc.), N-acyl glutamate, lauryl dimethylaminoacetic acid betaine, alkyl betaine, amidobetaine, sorbitan fatty acid esters (e.g., sorbitan monooleate, sorbitan monoisostearate, sorbitan monolaurate, sorbitan monopalmitate, sorbitan monostearate, sorbitan sesquioleate), glycerol polyglycerol fatty acid esters (e.g., glyceryl monoerucate, glyceryl sesquioleate, glyceryl monostearate, glyceryl monostearate malate, etc.), PEG-fatty acid esters (e.g., PEG-distearate, ethylene glycol distearate, etc.), PEG-alkyl ethers (e.g., PEG-2-octyldodecyl ether, etc.), sucrose fatty acid esters and the like. Their type and amount can be selected by those skilled in the art as required. Typically, the surfactant is present in the skin topical composition of the present invention in an amount of 0.01-50%, based on the total weight of the component (C).

The skin care active ingredients are known in the art and include, but are not limited to, humectant, skin conditioner, emollient and the like.

Examples of the humectant include, but are not limited to, one or more of glycerin, trehalose, sucrose, panthenol, propylene glycol, 1,2-pentanediol, mannitol, glycerin polyether-26, rhamnose, raffinose, erythritol, polyethylene glycol-8, polyethylene glycol-32, methyl glucitol polyether-10, methyl glucitol polyether-20, PEG/PPG-17/6 copolymer, sodium polyglutamate, xylitol, urea, hydrolyzed sclerotium gum, sodium polyglutamate, glyceryl glucoside, PPG-10 methyl glucose ether, pululan, tremellam, PPG-20 methyl glucose ether, and so on. Typically, the humectant is present in the skin topical composition of the present invention in an amount of 1-30%, based on the total weight of the component (C).

Examples of the emollient include, but are not limited to, glycerol triethylhexanoate, caprylic/capric triglyceride, grape seed oil, meadowfoam seed oil, butyrospermum parkii (shea butter), cetyl alcohol, polydimethyl siloxane, pentaerythrityl tetraethylhexanoate, olive oil, avocado oil, corn oil, dioctyl carbonate, homosalate, squalane, stearyl alcohol, isopropyl myristate, myristyl alcohol, hydrogenated polydecene, Mauritia Flexuosa fruit oil, sunflower seed oil, isohexadecane, jojoba oil, lanolin, paraffin, microcrystalline wax, beeswax and the like. Typically, the emollient is present in the skin topical composition of the present invention in an amount of 0.01-50%, based on the total weight of the component (C).

The skin conditioner can be used for moisturizing, anti-wrinkle, anti-freckle, anti-acne, oil control, etc. Examples include, but not limited to, one or more of phytosteryl/octyldodecyl lauroyl glutamate, curcuma longa, ceramide 2, ceramide 3, acetyl phytosphingosine, hydrolyzed sodium hyaluronate, acetyl phytosphingosine, cholesterol, kojic acid, ascorbic acid, ascorbyl glucoside, allantoin, birch bark extract, hydrogenated lecithin, arbutin, tranexamic acid, nicotinamide, acetyl phytosphingosine, protykin resveratrol, pterocarpus marsupium extract, plectranthus barbatus root extract, dragosantol, ascorbic acid tetraisopalmitate, pepper seed extract, ubiquinone, pyridoxine dipalmitate, pyridoxine dicaprylate, retinyl palmitate, tocopheryl acetate, Avena Sativa extract and the like. Typically, the skin conditioner is present in the skin topical composition of the present invention in an amount of 0.01-50%, based on the total weight of the component (C).

The pharmaceutical active ingredient is that known in the art for relieving skin itch. Examples include, but are not limited to, sophora flavescens extract, paeonol, linalool, cineote, borneol, sulfur, cicada, cuspid extract, scutella extract, bistort extract, perilla extract, calamine, sapnut saponin, portulaca oleracea extract, ammonium glycyrrhizinate, etc. Typically, the pharmaceutical active ingredient is present in the skin topical composition of the present invention in an amount of 0.01-80%, based on the total weight of the component (C).

The excipient includes, but is not limited to, an emulsifier, a thickener, a preservative, a perfume, a pH regulator and the like.

Examples of the emulsifier include, but are not limited to, one or more of sorbitan olivate, steareth-21, PEG-60 Hydrogenated castor oil, glycerol stearate/PEG-100 stearate, PPG-13-decyltetradeceth-24, cetearyl glucoside, polyglyceryl-10 myristate, cetearyl glucoside, polyglyceryl-10 stearate, polyglyceryl-10 dioleate, etc. Typically, the emulsifier is present in the skin topical composition of the present invention in an amount of 0.01-10%, based on the total weight of the component (C).

Examples of the thickener include, but are not limited to, one or more of hydroxyethyl cellulose, hydroxypropyl cellulose, carbomers, xanthan gum, gum arabic, polyethylene glycol-14M, polyethylene glycol-90M, succinyl polysaccharides, acrylates/C10-30 alkyl acrylate crosspolymer, etc. The type and amount thereof can be selected by those skilled in the art as required.

Examples of the preservative include, but are not limited to, one or more of methyl hydroxybenzoate, propyl hydroxybenzoate, phenoxyethanol, benzyl alcohol, phenylethanol, potassium sorbate, sodium benzoate, chlorophenesin, and other preservative synergists, such as pentanediol, hexanediol, caprylyl glycol, p-hydroxyacetophenone, etc. Typically, the preservative is present in the skin topical composition of the present invention in an amount of 0.01-10%, based on the total weight of the component (C).

Examples of the pH regulator include, but are not limited to, one or more of citric acid, sodium citrate, arginine, triethanolamine, etc. The type and amount thereof can be selected by those skilled in the art as required.

The skin topical composition according to the present invention can be prepared by any of suitable methods known in the art. For example, it can be prepared using a container commonly used in the art, such as a dissolving tank, an emulsifying pot, a disperser, a transfer pump. During the preparation, water-soluble substances are charged into an aqueous phase dissolving kettle, and oil-soluble substances are charged into an oil phase dissolving kettle, and the two kettles are heated to about 80°C, respectively; for agglomerated raw materials, they can be pre-dispersed by means of a disperser. Upon the completion of the dissolution, the oil phase and the aqueous phase are transferred into an emulsifying pot, and homogenized and emulsified for about 5-15mins. Upon the completion of the emulsification, the bulk is cooled to room temperature, and optionally a fragrance, a preservative and the like are added, and the pH of the product is adjusted as needed.

Above preparation methods can be varied or adjusted according to the dosage form requirements. Various dosage forms of pharmaceutical compositions or cosmetic compositions, such as liquid, lotion, ointment, cream, or gel can be prepared as needed, in which the cosmetic composition may be in various forms, such as lotion, spray, emuslion, stock solution, BB cream, sunscreen.

### Examples

The present invention is further described in detail below with reference to specific embodiments. It should be understood that the specific embodiments described herein are only used to explain the present invention in more detail and are not intended to limit the present invention. All similar substitutions and modifications will be apparent to those skilled in the art, and they are all considered to be included in the scope of the present invention.

### Example 1: Preparation of experimental and control samples

In this example, combination samples having the following formulation were prepared, wherein combinations A-1, A-2, and A-3 fall within the present invention, and combinations B-E are controls.

| | Comb*. A-1 ( wt% ) | Comb. A-2 ( wt% ) | Comb. A-3 ( wt% ) | Comb. B ( wt% ) | Comb. C ( wt% ) | Comb. D ( wt% ) | Comb. E (wt%) |
|---|---|---|---|---|---|---|---|
| Water | 0 | 0 | 0 | 0 | 50 | 90 | 50 |
| Betaine | 10 | 10 | 10 | 0 | 10 | 10 | 0 |
| Birch sap stock solution | 0 | 0 | 90 | 100 | 40 | 0 | 50 |
| 1.3 times concentrated birch sap | 90 | 0 | 0 | 0 | 0 | 0 | 0 |
| 3.5 times concentrated birch sap | 0 | 90 | 0 | 0 | 0 | 0 | 0 |

| | | | | | | | |
|---|---|---|---|---|---|---|---|
| *: comb.=combination | | | | | | | |

The preparation procedure of the above formulations was as follows: birch sap stock solution, concentrated birch sap or water were heated to 80°C, added with betaine, and stirred uniformly for later use. The resulting samples were all colorless and transparent solutions.

### Example 2: Effects of experimental and control samples on histamine release from RBL-2H3 cells

The effects of samples A-E as prepared above on the release of histamine from RBL-2H3 cells were tested according to the following test methods.

Experimental principle: Compound 48/80 (a polymer produced by condensation of N-methyl-p-methoxyphenylethylamine and formaldehyde) is a tool medicine to induce mast cell degranulation, the mechanism is that it acts on mast cell membrane and induces an increase of intracellular calcium ions to change the amount of second messengers cAMP and cGMP, resulting in mast cell degranulation and histamine release.

Experimental materials: RBL-2H3 cell line was provided by the Animal Center of Zhejiang Academy of Medical Sciences; the test samples included above seven samples combinations; the reagents included fetal bovine serum, Tyrode's salt, Compound 48/80, and histamine Elisa kit.

### Test steps:

| | |
|---|---|
| Preparation of Compound 48/80 solution | 1.0 mg of 48/80 powder was weighed and diluted with 1 mL of Tyrode's solution, and 50 uL of the dilution was diluted to 1000 uL, i.e. Compound 48/80 solution at a concentration of 50 ug/mL, and use solution right after it was ready. |
| Preparation of Tyrode's solution | One part of Tyrode's salt was diluted with 80 mL of sterilized deionized water and volumed to 100 mL, thereby preparing Tyrode's solution of 10 times concentration, and autoclave sterilized. |
| Cell treatment | RBL-2H3 cells in logarithmic growth phase were spread to the bottom of a culture flask and digested with a digestive solution containing 0.25% trypsin and 0.03% EDTA, centrifuged at 1200 rpm for 5mins; resuspended with 1 times Tyrode's solution and counted, and diluted to 1×10⁴/mL; centrifuged again at 1200 rpm for 5mins to remove the supernatant. |
| Sample grouping and experiments | Model group: 700 uL of cell suspension was centrifuged and resuspended with Tyrode's solution, held at 37°C for 30mins; sample group: 700 uL of cell suspension was centrifuged and resuspended with sample solution, held at 37°C for 30mins; the samples were mixed well and dispensed into 3 tubes, 200 uL per |
| | tube; the model group and the 7 sample groups: 50 uL of Compound 48/80 solution was added to the cell suspension to reach a final concentration of 10 ug/mL, and held at 37°C for 20mins. 3 replicates in each group. |
| Histamine determination | The above groups of samples were cooled in an ice box for 10mins to stop the reaction. The samples after the reaction were centrifuged (4°C, 1500 rpm, 30mins) to precipitate the cells at the bottom of EP tube, and the supernatants were transferred to new centrifuge tubes. The precipitated cells were resuspended with 250 µL of 1 times Tyrode's solution, heated in a 90°C water bath, taken out after 5mins and immediately placed in an ice box, after the samples were completely cooled, they were taken out and heated again in a 90°C water bath, and frozen-thawed repeatedly several times to break cells. The supernatants and the cell solutions were diluted 10 times, and the stock solutions were stored in a refrigerator at -20°C. The diluted samples were determined for histamine using an imported histamine kit. The histamine release was calculated using the following formula: histamine release (%) = extracellular histamine release amount/(extracellular histamine release amount + intracellular histamine amount) × 100% |

Experimental results: The experimental results are summarized in Table 1 below.

**Table 1: Test results of histamine release in cell model**

| | Release% | P value (vs. model) | P value (vs. Combination A-1) |
|---|---|---|---|
| Model group | 72.7±3.2 | - | 0.000 |
| Combination A-1 | 30.9±1.1 | 0.000 | - |
| Combination A-2 | 36.3±1.6 | 0.000 | 0.003 |
| Combination A-3 | 40.4±1.0 | 0.000 | 0.009 |
| Combination B | 45.1±2.9 | 0.000 | 0.004 |
| Combination C | 51.8±3.9 | 0.001 | 0.001 |
| Combination D | 57.5±4.7 | 0.004 | 0.001 |
| Combination E | 59.9±2.8 | 0.003 | 0.000 |

The above results of the cell experiments showed that combinations A-1, A-2 and A-3 within the scope of the present invention significantly reduced histamine release amount as compared to control combinations B-E. This showed that the combination of birch sap and betaine or the combination of concentrated birch sap and betaine had synergistic effects on inhibition of histamine release, and the combination of 1.3 times concentrated birch sap and betaine showed further better effects.

### Example 3: Effect of experimental and control samples on release of interleukin-4 (IL-4) from mast cells

According to the following test method, the effects of the seven samples A-E as prepared above on the release of IL-4 from mast cells were tested.

Experimental principle: The specific antigen is crosslinked with the IgE antibody bound to the cell membrane, resulting in cell activation and rapid secretion of inflammatory mediators related to granulation (histamine, IL-4, IL-13, etc.). In addition to immune triggering, the degranulation reaction of mast cells can be caused by non-specific triggering agents such as anti-IgE, Compound 48/80, etc., these agents exhibit characteristics similar to those that rely on IgE release mediators. In this study, Compound 48/80 was used to stimulate mast cells to degranulate and release the inflammatory mediator IL-4 to investigate the protective efficacy of the test samples on the allergic reaction of mast cells.

Experimental materials: Mast cells were provided by the Animal Center of Zhejiang Academy of Medical Sciences. Reagents included fetal bovine serum, RPM1640 medium, Tyrode's salt, Compound 48/80, sodium cromoglycate, and Elisa (interleukin-4) kit. The test samples included control group, model group, positive drug (sodium cromoglycate) group, and above seven samples A-E.

Experimental method: Each of samples was prepared using RPMI1640 medium, and each of samples was provided with 8 duplicate wells. Mast cells were inoculated into a 96-well culture plate at a density of 5×10⁶/mL, 90 µL per well, and added with 10 µL/well of a corresponding sample; cultured in a 5% CO₂ incubator at 37°C, and taken out after 4 h; in addition to the control group, the other groups were added with a stimulus 48/80 (final concentration 20 mg/L), and continued to incubate in an incubator at 37°C for 2h, and then centrifuged at 1500 rpm for 5 mins to get the supernatant., and the content of IL-4 in the supernatant was detected by Elisa (interleukin-4) kit.

Experimental results: The experimental results are summarized in Table 2 below.

**Table 2: Test results of IL-4 release from cell models**

| | Release% | P value(vs model) |
|---|---|---|
| Control group | 34.4±7.9 | 0.000 |
| Model group | 67.6±9.9 | - |
| Positive drug group | 44.2±12.3 | 0.003 |
| Combination A-1 | 50.8±14.7 | 0.014 |
| Combination A-2 | 52.9±15.7 | 0.035 |
| Combination A-3 | 53.2±11.3 | 0.031 |
| Combination B | 55.7±12.5 | 0.043 |
| Combination C | 57.6±15.0 | 0.083 |
| Combination D | 61.4±12.1 | 0.109 |
| Combination E | 61.6±9.1 | 0.132 |

The results showed that the combinations A-1, A-2 and A-3 within the scope of the present invention all significantly inhibited (p<0.05) IL-4 release caused by Compound 48/80 stimulating mast cells. This showed that the combination of birch sap and betaine or the combination of concentrated birch sap and betaine has a synergistic effect on the inhibition of IL-4 release, and the combination of 1.3 times concentrated birch sap and betaine showed further better effects.

### Example 4: Preparation of spray composition having antipruritic efficacy This example provided a spray composition having an antipruritic efficacy, and its formulation was as follows:

| Ingredients | Amount (wt% ) |
|---|---|
| Birch sap | 77.75 |
| Betaine | 5 |
| Glycerin | 5 |
| Glycerin polyether-26 | 0.5 |
| Phytosteryl/octyldodecyl lauroyl glutamate | 1 |
| Butanediol | 5 |
| Pentanediol | 2 |
| Glycerol triethylhexanoate | 2 |
| PEG-60 hydrogenated castor oil | 1 |
| Citric acid | 0.1 |
| Arginine | 0.1 |
| Methyl hydroxybenzoate | 0.15 |
| Phenoxyethanol | 0.4 |

The above spray composition was prepared as follows:
1. Birch sap was heated to 80°C, added with betaine, citric acid, glycerin, methyl hydroxybenzoate, butanedio and pentanediol in turn and mixed, stirred to dissolve uniformly;
2. Phytosteryl/octyldodecyl lauroyl glutamate, glycerol triethylhexanoate, glycerin polyether-26, and PEG-60 hydrogenated castor oil were heated to 80°C, stirred uniformly, and then added with the mixture obtained in 1;
3. The resultant was cooled to 40°C, added with phenoxyethanol, and then the pH was adjusted with arginine, and discharged.

Efficacy test: 30 patients over 40 years old with pruritus senilis, male or female, only itch and no skin lesions were selected. The above spray composition was applied to the itch site after cleaning every morning and evening. Before and 4 weeks after the use of the composition, self-evaluation was carried out for itch and was ranked into significant improvement, obvious improvement, certain improvement, no significant improvement, and no improvement depending on the degree of improvement. The results showed that 27 patients (90%) had a significant improvement in itch after using the product, while the other 3 patients also had certain improvement. This showed that the spray composition had an excellent antipruritic efficacy.

### Example 5: Preparation of lotion composition having antipruritic efficacy This example provided a lotion composition having an antipruritic efficacy, and its formulation was as follows:

| Ingredients | Amount (wt% ) |
|---|---|
| Birch sap | 76.7 |
| Betaine | 6 |
| Allantoin | 0.4 |
| Panthenol | 0.6 |
| Glycerin | 5 |
| Hydrolyzed sodium hyaluronate | 0.5 |
| Pentanediol | 3 |
| Caprylic/capric triglycerides | 3 |
| Butyrospermum parkii (shea butter) | 1 |
| Carbomer | 0.1 |
| Xanthan gum | 0.1 |
| Cetyl alcohol | 1 |
| Glycerol stearate/PEG-100 stearate | 1 |
| Glycerol triethylhexanoate | 1 |
| Phenoxyethanol | 0.4 |
| Methyl hydroxybenzoate | 0.1 |
| Arginine | 0.1 |

The above lotion composition was prepared as follows:
1. Aqueous phase: birch sap, glycerin, betaine, panthenol, allantoin, hydrolyzed sodium hyaluronate, pentanediol, xanthan gum, carbomer and methyl hydroxybenzoate were mixed and heated to 80°C, stirred to dissolve uniformly;
2. Oil phase: cetyl alcohol, glycerol stearate/PEG-100 stearate, caprylic/capric triglyceride, glycerol triethylhexanoate, butyrospermum parkii (shea butter). The above raw materials were heated to 80°C, and stirred to dissolve uniformly;
3. The aqueous phase and the oil phase were mixed, homogenized and emulsified for 5mins. Upon the completion of the emulsification, arginine was added, stirred for 15mins, cooled to 40°C, and phenoxyethanol was added.

Efficacy test: 30 patients over 40 years old with pruritus, male or female, only itch and no skin lesions were selected. The above lotion composition was applied to the itch site after cleaning every morning and evening. Before and 4 weeks after the use of the composition, self-evaluation was carried out for itch and was ranked into significant improvement, obvious improvement, certain improvement, no significant improvement, and no improvement depending on the degree of improvement. The results showed that 28 patients (93.3%) had a significant improvement in itch after using the product, while the other 2 patients also had certain improvement. This showed that the lotion composition had an excellent antipruritic efficacy.

### Example 6: Preparation of body milk composition having antipruritic efficacy

This example provided a body milk composition having an antipruritic efficacy, and its formulation was as follows:

| Ingredients | Lotion A (wt% ) |
|---|---|
| Birch sap | 70.03 |
| Betaine | 3 |
| Allantoin | 0.1 |
| Panthenol | 0.6 |
| Glycerin | 8 |
| Hydrolyzed sodium hyaluronate | 0.1 |
| Trehalose | 2 |
| Polymethylsilsesquioxane | 1 |
| Myristyl alcohol | 1 |
| Beeswax | 0.2 |
| Stearyl alcohol | 0.3 |
| Stearic acid | 0.5 |
| Polydimethylsiloxane | 1 |
| Nicotinamide | 0.3 |
| Triethanolamine | 0.2 |
| Acrylates/C10-30 alkyl acrylate | 0.15 |
| crosspolymer | |
| Dipropylene glycol | 3 |
| Caprylic/capric triglycerides | 3 |
| Butyrospermum parkii (shea butter) | 2 |
| Carbomer | 0.1 |
| Xanthan gum | 0.1 |
| Hydrogenated lecithin | 0.3 |
| Glycerol stearate/PEG-100 stearate | 1 |
| Caprylyl glycol | 0.02 |
| Phenoxyethanol | 0.4 |
| Methyl hydroxybenzoate | 0.1 |
| Avena Sativa extract | 1.5 |

The above body milk composition was prepared as follows:
1. Aqueous phase: birch sap, glycerin, betaine, panthenol, allantoin, hydrolyzed sodium hyaluronate, dipropylene glycol, trehalose, hydrogenated lecithin, xanthan gum, carbomer, acrylates/C10-30 alkyl acrylate crosspolymer and methyl hydroxybenzoate were mixed and heated to 80°C, stirred to dissolve uniformly;
2. Oil phase: glycerol stearate/PEG-100 stearate, caprylic/capric triglyceride, polymethylsilsesquioxane, myristyl alcohol, beeswax, stearyl alcohol, stearic acid, butyrospermum parkii (shea butter), polydimethylsiloxane. The above raw materials were heated to 80°C, stirred to dissolve uniformly;
3. The aqueous phase and the oil phase were mixed, homogenized and emulsified for 5mins. Upon the completion of the emulsification, triethanolamine was added, stirred for 15mins, cooled to 40°C, and phenoxyethanol, Avena Sativa extract, caprylyl glycol and nicotinamide were added.

Efficacy test: 30 patients over 40 years old with pruritus, male or female, only itch and no skin lesions were selected. The above lotion composition was applied to the itch site after cleaning every morning and evening. Before and 4 weeks after the use of the composition, self-evaluation was carried out for itch and was ranked into significant improvement, obvious improvement, certain improvement, no significant improvement, and no improvement depending on the degree of improvement. The results showed that 29 patients (96.7%) had a significant improvement in itch after using the product, while the other 1 patient also had an obvious improvement. This showed that the body milk composition had an excellent antipruritic efficacy.

### Example 7: Preparation of cream composition having antipruritic efficacy

This example provided a cream composition having an antipruritic efficacy, and its formulation was as follows:

| Ingredients | Amount (wt% ) |
|---|---|
| Birch sap | 76.95 |
| Betaine | 3 |
| Allantoin | 0.1 |
| Panthenol | 0.5 |
| Glycerin | 5 |
| Hydrolyzed sodium hyaluronate | 0.5 |
| Glycerin polyether-26 | 2 |
| Xanthan gum | 0.1 |
| Glyceryl caprylate/caprate | 2 |
| Butyrospermum parkii (shea butter) | 1 |
| Cetyl alcohol | 1 |
| Glycerol stearate/PEG-100 stearate | 1.5 |
| Pentaerythrityl tetraethylhexanoate | 3 |
| Polydimethylsiloxane | 2 |
| Methyl hydroxybenzoate | 0.2 |
| Propyl hydroxybenzoate | 0.1 |
| Carbomer | 0.3 |
| Phenoxyethanol | 0.4 |
| Caprylyl glycol | 0.05 |
| Arginine | 0.3 |

The above cream composition was prepared as follows:
1. Aqueous phase: birch sap, betaine, glycerin, panthenol, allantoin, hydrolyzed sodium hyaluronate, glycerin polyether-26, xanthan gum, carbomer, methyl hydroxybenzoate;
2. Oil phase: cetyl alcohol, glyceryl caprylate/caprate, pentaerythrityl tetraethylhexanoate, glycerol stearate/PEG-100 stearate, butyrospermum parkii (shea butter, polydimethylsiloxane, propyl hydroxybenzoate. The above raw materials were heated to 80°C, stirred to dissolve uniformly;
3. The aqueous phase and the oil phase were mixed, homogenized and emulsified for 5mins. Upon the completion of the emulsification, Arginine was added, stirred for 15mins, cooled to 40°C, and phenoxyethanol and caprylyl glycol were added in turn.

Efficacy test: 30 patients over 40 years old with pruritus, male or female, only itch and no skin lesions were selected. The above lotion composition was applied to the itch site after cleaning every morning and evening. Before and 4 weeks after the use of the composition, self-evaluation was carried out for itch and was ranked into significant improvement, obvious improvement, certain improvement, no significant improvement, and no improvement depending on the degree of improvement. The results showed that 28 patients (93.3%) had a significant improvement in itch after using the product, while the other 2 patient also had an obvious improvement. This showed that the cream lotion composition had an excellent antipruritic efficacy.

The technical solutions of the above-mentioned examples are preferred embodiments of the present invention, and do not constitute a limitation to the scope of the appended claims. Various modifications and variations can be made without departing from the spirit and principle of the present invention, and these modifications and variations should also be considered within the scope of the present invention.

## Claims

1. Use of a combination of betaine and birch sap in a skin topical composition having an antipruritic efficacy, wherein the skin topical composition does not comprise water added as a separate component.

2. The use according to claim 1, wherein the birch sap is birch sap stock solution.

3. The use according to claim 1, wherein the birch sap is a concentrated birch sap having a concentration degree of 1.05-8 times, preferably 1.1-4 times, more preferably 1.2-2 times.

4. The use according to any one of claims 1-3, wherein the skin topical composition is a pharmaceutical composition or a cosmetic composition.

5. A skin topical composition having an antipruritic efficacy, comprising a combination of (A) birch sap and (B) betaine, wherein the skin topical composition does not comprise water added as a separate component.

6. The composition according to claim 5, wherein the birch sap is birch sap stock solution.

7. The composition according to claim 5, wherein the birch sap is a concentrated birch sap having a concentration degree of 1.05-8 times, preferably 1.1-4 times, more preferably 1.2-2 times.

8. The composition according to any one of claims 5-7, which is a pharmaceutical composition or a cosmetic composition.

9. The composition according to any one of claims 5-8, which comprises 5-99.9% by weight, preferably 20-95% by weight, more preferably 35-90% by weight of birch sap, based on the total weight of the skin topical composition.

10. The composition according to any one of claims 5-9, which comprises 0.01-20% by weight, preferably 0.1-15% by weight, more preferably 1-10% by weight of betaine, based on the total weight of the skin topical composition.

## Amended claims

### Amended claims under Art. 19.1 PCT

1. Use of a combination of betaine and birch sap in a skin topical composition having an antipruritic efficacy, wherein the skin topical composition does not comprise water added as a separate component.

2. The use according to claim 1, wherein the birch sap is birch sap stock solution.

3. The use according to claim 1, wherein the birch sap is a concentrated birch sap having a concentration degree of 1.05-8 times, preferably 1.1-4 times, more preferably 1.2-2 times.

4. The use according to any one of claims 1-3, wherein the skin topical composition is a pharmaceutical composition or a cosmetic composition.

5. A skin topical composition having an antipruritic efficacy, comprising a combination of (A) birch sap and (B) betaine, wherein the skin topical composition does not comprise water added as a separate component, wherein the birch sap is a concentrated birch sap having a concentration degree of 1.05-8 times, preferably 1.1-4 times, more preferably 1.2-2 times.

6. The composition according to claim 5, which is a pharmaceutical composition or a cosmetic composition.

7. The composition according to any one of claims 5-6, which comprises 5-99.9% by weight, preferably 20-95% by weight, more preferably 35-90% by weight of birch sap, based on the total weight of the skin topical composition.

8. The composition according to any one of claims 5-7, which comprises 0.01-20% by weight, preferably 0.1-15% by weight, more preferably 1-10% by weight of betaine, based on the total weight of the skin topical composition.

9. A cosmetic method for providing antipruritic efficacy to skin, comprising applying to the skin a skin topical composition having an antipruritic efficacy, wherein the skin topical composition comprises a combination of (A) birch sap and (B) betaine, but does not comprise water added as a separate component.

10. The cosmetic method according to claim 9, wherein the birch sap is birch sap stock solution.

11. The cosmetic method according to claim 9, wherein the birch sap is a concentrated birch sap having a concentration degree of 1.05-8 times, preferably 1.1-4 times, more preferably 1.2-2 times.

12. The cosmetic method according to any one of claims 9-11, wherein the skin topical composition comprises 5-99.9% by weight, preferably 20-95% by weight, more preferably 35-90% by weight of birch sap, based on the total weight of the skin topical composition.

13. The cosmetic method according to any one of claims 9-12, which comprises 0.01-20% by weight, preferably 0.1-15% by weight, more preferably 1-10% by weight of betaine, based on the total weight of the skin topical composition.
